# EUROPÄISCHE PATENTSCHRIFT

(11) **EP 3 706 834 B1**
(45) Veröffentlichungstag und Bekanntmachung des Hinweises auf die Patenterteilung: **08.07.2026**
(21) Anmeldenummer: 18822133.7
(22) Anmeldetag: 09.11.2018
(51) Int. Cl.: A61M 5/30, A61M 5/24, A61M 5/315

(54) **VERFAHREN UND VORRICHTUNG ZUM NADELLOSEN INJIZIEREN VON FLÜSSIGKEIT IN EIN SUBSTRAT**
METHOD AND DEVICE FOR THE NEEDLE-FREE INJECTING OF FLUID INTO A SUBSTRATE
PROCÉDÉ ET DISPOSITIF D'INJECTION SANS AIGUILLE D'UN LIQUIDE DANS UN SUBSTRAT

(30) Priorität: 10.11.2017 DE 102017126493
(43) Veröffentlichungstag der Anmeldung: 16.09.2020
(73) Patentinhaber: Conzima GmbH, 87487 Wiggensbach (DE)
(72) Erfinder: SCHMITT, Fritz, 6832 Betzdorf (LU)
(74) Vertreter: Angerhausen, Christoph
(86) Internationale Anmeldenummer: PCT/IB2018/058813
(87) Internationale Veröffentlichungsnummer: WO 2019/092644

(56) Entgegenhaltungen:
- WO-A1-2016/102407
- US-A1- 2010 016 827
- US-A1- 2017 312 435
- US-B1- 6 224 567

## Beschreibung

Die Erfindung ist ferner auf eine Injektionsvorrichtung zum nadellosen Injizieren von Flüssigkeit in ein Substrat gerichtet, insbesondere eines flüssigen, pharmazeutischen oder kosmetischen Präparats in ein biologisches Gewebe, mit einem Gehäuse zur Aufnahme eines die Flüssigkeit aufnehmenden Flüssigkeitsbehältnisses und mit einer Ejektoreinrichtung zum Ausstoßen der Flüssigkeit aus dem Flüssigkeitsbehältnis durch eine Auslassdüse. Eine derartige Vorrichtung ist aus der US 2010/016827 A1 und der US 6 224 567 B1 bekannt. Um eine Flüssigkeit in ein Substrat zu injizieren, beispielsweise ein flüssiges pharmazeutisches oder kosmetisches Präparat in oder unter die Haut eines Menschen oder anderen Lebewesens, wird die Flüssigkeit regelmäßig durch eine Injektionsnadel in das Substrat, also das menschliche oder tierische Gewebe eingeleitet. Die Injektionsnadel muss hierzu zunächst in das Substrat eingestochen werden. Infolge des dabei mittels einer Schneide an der Nadelspitze erzeugten Einschnitts kommt es zu Verletzungen, die in lebendem Gewebe zwar meist schnell wieder verheilen, aber regelmäßig eine Narbenbildung zur Folge haben. Außerdem bergen Injektionen mit Injektionsnadeln immer das Risiko einer Infektion. Eine derartige Vorrichtung ist aus der US 2017/312435 A1 bekannt. Eine ähnliche Vorrichtung beschreibt auch die WO 2016/102407 A1.

Es hat daher in der Vergangenheit verschiedene Versuche mit hypodermischen Strahlinjektionsvorrichtungen für die nadellose Injektion gegeben, um unter Verzicht der Verwendung einer in das Substrat einstechbaren Injektionsnadel eine kleine Menge Flüssigkeit wie z.B. einen Impfstoff oder ein anderes Arzneimittel, ein Anästhetikum oder dergleichen direkt durch die Hautoberfläche in das Gewebe einzubringen. Grundsätzlich bestand die Idee bei diesen Anstrengungen darin,
die Haut des Patienten alleine durch den Druck der Flüssigkeit zu durchdringen und das Injektionsmedium in eine gewünschte Tiefe zu bringen. Die meisten zu diesem Zweck entwickelten Geräte konnten allerdings die in sie gesetzten Erwartungen entweder gar nicht oder jedenfalls nicht zufriedenstellend erfüllen.

Die in der Vergangenheit zur nadellosen Injektion von Flüssigkeiten wie beispielsweise Arzneimitteln vorgeschlagenen Injektionsgeräte haben einen Energiespeicher wie beispielsweise einen Federspeicher, einen Druckspeicher und/oder eine Zündkapsel, derbeim Auslösen eine Druckerhöhung in einem im Gerät enthaltenen Flüssigkeitsvorrat bewirkt, um aus dem Vorrat Flüssigkeit durch eine Auslassdüse auszustoßen. Der Düsenquerschnitt ist dabei möglichst klein und der auf den Flüssigkeitsvorrat wirkende Druck möglichst hoch, um einen Flüssigkeitsstrahl mit geringem Querschnitt und großer Strahlgeschwindigkeit zu erzeugen. Aufgrund des bei Auslösen des Energiespeichers in dem Flüssigkeitsvorrat sehr plötzlich auftretenden, großen statischen Druckanstiegs kann es leicht zu einer unerwünschten Veränderung und/oder Beschädigung der Flüssigkeit kommen, z.B. dadurch, dass infolge der plötzlichen starken Druckerhöhung in der Flüssigkeit es zum Aufbrechen von Molekülen von in der Flüssigkeit enthaltenen, pharmazeutischen Wirkstoffen kommt, die dadurch in ihrer Wirksamkeit verändert werden oder diese vollständig verlieren, ohne dass dies bei der Injektion durch die Haut eines Patienten feststellbar wäre. Es ist dann unklar, ob und in welchem Umfang ein vorher in bekannter Menge in dem Flüssigkeitsbehältnis vorhandener Wirkstoff nach dem Injektionsvorgang in das Gewebe tatsächlich seine vorgesehene Wirkung entfalten kann.

Aus der US 2002/0143323 A1 ist eine endoskopische Einrichtung zur gastrointestinalen Epithel-Entfernung bekannt, bei der eine Sonde mit einer Flüssigkeit versorgt wird. Die Flüssigkeit wird der Sonde aus einem Vorratsbehälter zugeführt, der mit unter Druck stehendem Gas aus einer Gasflasche beaufschlagbar ist. Die US 2006/0149193 A1 offenbart eine Einrichtung mit einer Sonde und einem Flüssigkeitsapplikator, der eine Flüssigkeitsaustrittsöffnung zur nadellosen Injektion einer Flüssigkeit in ein biologisches Gewebe und eine zu der Flüssigkeitsaustrittsöffnung führende Flüssigkeitsleitung aufweist. Eine zugeordnete Flüssigkeitsliefereinrichtung weist eine Antriebseinrichtung auf und ist mit einem druckspeichernden Druckbehälter als Energiespeicher verbindbar. Zu der Flüssigkeitsliefereinrichtung gehört eine Expansionskammer, die eine bewegliche Wandfläche aufweist, die die zu injizierende Flüssigkeit umschließt und die mit einer Druckflüssigkeit beaufschlagbar ist.

Weiter sind Geräte bekannt, die zur nadellosen Injektion einer Flüssigkeit unter die Mucosa dienen. Zum Beispiel offenbart die US 2009/0157114 AI ein Endoskop mit einer Sonde zur nadellosen Unterspritzung der Mucosa. Die Sonde stößt dazu einen Strahl Natriumchloridlösung aus, der aufgrund seines geringen Querschnitts und seiner gleichzeitig hohen Geschwindigkeit in das Gewebe eindringt. Um die Natriumchloridlösung zu fördern und den entsprechenden Druck zu erzeugen, ist eine Pumpeinheit oder gegebenenfalls ein kraftverstärkender Hebel vorgesehen.

Aufgabe der Erfindung ist es daher, eine Vorrichtung der eingangs genannten Art zu schaffen, womit die Flüssigkeit beim Injektionsvorgang möglichst schonend behandelt wird. Diese Aufgabe wird durch eine Vorrichtung nach Anspruch 1 gelöst. Die abhängigen Ansprüche betreffen vorteilhafte Ausführungsformen. Die erfindungsgemäße Vorrichtung ermöglicht ein Verfahren, bei dem das Flüssigkeitsbehältnis gemeinsam mit der darin aufgenommenen Flüssigkeit auf eine Anfangsgeschwindigkeit beschleunigt wird, bevor die Flüssigkeit aus dem Flüssigkeitsbehältnis durch die Auslassdüse ausgestoßen wird. In vorteilhafter Ausgestaltung wird dann die Bewegung des Flüssigkeitsbehältnisses nach Beschleunigung auf die Anfangsgeschwindigkeit abgebrochen, während zumindest eine Teilmenge der darin aufgenommenen Flüssigkeit weiter bewegt und durch die Auslassdüse ausgestoßen wird. Die Flüssigkeit bzw. deren Teilmenge bewegt sich hat also bei Beginn ihres Ausstoßes aus dem Flüssigkeitsbehältnis bereits selbst mit einer Ausstoßgeschwindigkeit, hat also zu diesem Zeitpunkt bereits eine kinetische Druckkomponente, um die sich die (statische) Druckerhöhung reduziert, zu der es in dem Gesamtsystem während des Injektionsvorgangs kommen kann. Das Maß der statischen Druckerhöhung in der Flüssigkeit hängt im Wesentlichen nur noch von der Form und dem Grad der Querschnittsverengung ab, den die ausgestoßene Flüssigkeit bei ihrem Weg aus dem Flüssigkeitsbehältnis durch die Auslassdüse durchläuft, also von dem Strömungswiderstand, den die Auslassdüse der durch sie ausgestoßenen Flüssigkeit entgegensetzt. In vorteilhafter Ausgestaltung der Erfindung kann der Flüssigkeitsstrahl vor und/oder während seines Durchgangs durch die Auslassdüse zumindest an seinem Außenumfang in Rotation um seine Flüssigkeitsstrahlachse versetzt werden, wodurch. ein Aufweiten, also eine Querschnittsvergrößerung, des Flüssigkeitsstrahls auf seinem Weg vom Auslass der Düse zur Substratoberfläche und das bei den bekannten Vorrichtungen immer wieder beobachtete Aufpilzen beim Auftreffen auf das Substrat sehr zuverlässig vermieden wird. Es wird angenommen, dass infolge der Rotation wirkende Zentripetalkräfte die Flüssigkeitsteilchen (Moleküle) zusammenhalten, und zwar nicht nur auf dem Weg des Flüssigkeitsstrahls von der Auslassdüse zur Substratoberfläche, sondern auch beim Eindringen in das Substrat. Tatsächlich scheint, jedenfalls bei geeigneter Gestaltung der Auslassdüse, es durch die Rotation des Strahls nach dessen Austritt aus der Auslassdüse sogar zu einer Querschnittsverringerung und damit zu einer Geschwindigkeitserhöhung des Flüssigkeitsstrahls zu kommen, so dass dieser auf das Substrat sogar mit einer größeren Geschwindigkeit auftreffen kann, als er beim Austritt aus einer Auslassdüse aufweist. Versuche haben gezeigt, dass der Flüssigkeitsstrahl bei einer Injektion in biologisches Gewebe wie z.B. in oder unter die Haut eines Menschen oder eines Tieres zuverlässig in dieses eindringt, auch wenn die Auslassdüse der erfindungsgemäßen Vorrichtung in einem Abstand von der Gewebeoberfläche positioniert ist, der Flüssigkeitsstrahl also die Distanz zwischen der Düse und der Gewebeoberfläche als "Freistrahl" zu überbrücken hat, und zwar ohne dass eine Vergrößerung der Distanz einen nachteiligen Einfluss auf die Injektionsqualität haben würde. Die Rotation, die dem Strahl vor seinem Auftreffen auf das Substrat aufgeprägt wird, überlagert sich mit der translatorischen Bewegung der Flüssigkeit in dessen Strahlrichtung zu einer schraubenförmigen Bewegung, mit der sich der Strahl nach den gemachten Beobachtungen mit sehr geringem Widerstand an der Oberfläche des Substrats in dieses unter Ausbildung eines dem Strahlquerschnitt entsprechenden Einlasskanals praktisch "hineinbohrt" oder "hineinschraubt", wobei tatsächlich praktisch keine der auf das Substrat auftreffenden Flüssigkeit verlorengeht, also nicht mit in das Substrat eindringt.

Die erfindungsgemäße Injektionsvorrichtung der eingangs genannten Art, mit der das vorstehend erläuterte Verfahren nach der Erfindung ausgeführt werden kann, zeichnet sich aus durch eine Beschleunigungseinrichtung zum Beschleunigen des Flüssigkeitsbehältnisses mit der darin aufgenommenen Flüssigkeit auf eine Anfangsgeschwindigkeit im Inneren des Gehäuses und eine Verzögerungseinrichtung zum Abbrechen der Bewegung des Flüssigkeitsbehältnisses. Mittels der Beschleunigungseinrichtung wird zunächst das Flüssigkeitsbehältnis einschließlich der darin aufgenommenen Flüssigkeit auf die Anfangsgeschwindigkeit beschleunigt, bevor die Verzögerungseinrichtung die Bewegung des Flüssigkeitsbehältnisses, beispielsweise durch einen zwischen dem Gehäuse und dem Flüssigkeitsbehältnis wirksamen Anschlag, wieder abbremst, während allerdings die Bewegung der im Behältnis enthaltenen Flüssigkeit zumindest teilweise aufrecht erhalten bleibt, so dass die Flüssigkeit das Flüssigkeitsbehältnis verlässt und durch die Auslassdüse austritt. Der Anschlag, der Bestandteil der Verzögerungseinrichtung ist bzw. diese im Wesentlichen bildet, kann mit einem Anschlagdämpfer, beispielsweise einem Elastomer-Puffer Element versehen sein. Auf dieser Weise erfolgt die Abbremsung des Flüssigkeitsbehältnisses nicht schlagartig, sondern entsprechend dem Dämpfungsvermögen des Anschlagdämpfers, womit Beschädigungen des Behältnisses, das ggf. aus einem zerbrechlichen Material wie Glas bestehen kann, sicher vermieden werden.

**In** vorteilhafter Weiterbildung der Erfindung ist vorgesehen, dass das Flüssigkeitsbehältnis einen die Flüssigkeit aufnehmenden Zylinderraum aufweist, in den das Druckstück einschiebbar ist. Das Flüssigkeitsbehältnis kann somit nach Art einer Zylinderampulle (Karpule) ausgestaltet sein, wie sie bei Zylinderampullenspritzen beispielsweise in der Dentalmedizin zum Einsatz kommt. Das Flüssigkeitsbehältnis kann einen an einen Einlassquerschnitt der Auslassdüse angepassten Behälterauslass aufweisen. Es ist in besonders vorteilhafter Weise möglich, dass das Flüssigkeitsbehältnis an seiner Auslassseite mit einem Durchstechstopfen verschlossen ist, der von einer vorzugsweise einlassseitig an der Auslassdüse vorgesehenen Hohlnadel zur Öffnung des Flüssigkeitsbehältnisses durchstechbar ist, und zwar zu einem Zeitpunkt, nachdem das Flüssigkeitsbehältnis zusammen mit der darin aufgenommenen Flüssigkeit auf die Anfangsgeschwindigkeit beschleunigt wurde, also insbesondere dann, wenn die
Bewegung des Flüssigkeitsbehältnisses wieder abgebrochen wird. Durch die dann erfolgende Öffnung der Zylinderampulle (Flüssigkeitsbehältnis) beim Durchstechen des Durchstechstopfens mit der Hohlnadel kann die Flüssigkeit aus dem Flüssigkeitsbehältnis durch die Hohlnadel hindurch weiter bewegt werden (durchströmen) und durch die auslassseitig der Hohlnadel anschließende Auslassdüse ausgestoßen werden. Es ist auch möglich, dass die Hohlnadel einstückig mit der Auslassdüse ausgestaltet ist bzw. diese selbst bildet.

Der Behälterauslass kann eine vorzugsweise vom Querschnitt des Zylinderraums hin zum Einlassquerschnitt der Auslassdüse konvergierende Beschleunigungszone aufweisen, wodurch die Flüssigkeit bei Ihrem Austritt aus dem Flüssigkeitsbehältnis weiter beschleunigt wird. Wenn die Auslassdüse in besonders vorteilhafter Weise integral oder auswechselbar am Flüssigkeitsbehältnis angeordnet oder anordbar ist, macht sie dessen Bewegung und Verzögerung während des Injektionsvorganges mit.

Die Ejektoreinrichtung weist erfindungsgemäß einen elektromagnetisch, chemisch und/oder Gas betriebenen Antrieb auf. Als besonders gut geeignet hat sich ein elektromagnetischer Antrieb erwiesen, mit dem es möglich ist, das Flüssigkeitsbehältnis zusammen mit der darin enthaltenen Flüssigkeit innerhalb kürzester Zeit und damit auf nur kurzer Strecke auf eine sehr hohe Geschwindigkeit zu beschleunigen, bevor das Flüssigkeitsbehältnis dann von der Verzögerungseinrichtung wieder abgebremst wird, während die mitbeschleunigte Flüssigkeit (oder jedenfalls eine Teilmenge der Flüssigkeit) das dann an seinem Auslass offene Behältnis ohne nennenswerten (statischen) Druckanstieg verlässt, indem sie einfach ihre Bewegung fortsetzt. Mittels eines elektomagnetischen Antriebs ist es auch in vorteilhafter Weise möglich, das Flüssigkeitsbehältnis nicht nur in axialer Richtung translatorisch zu bewegen, sondern auch, es in Rotation zu versetzen und damit zu bewirken, dass der Flüssigkeitsstrahl bei seinem Austritt aus der Auslassdüse diese mit der vorstehend bereits beschriebenen Rotation um seine Strahlachse verlässt.

Erfindungsgemäß ist das Flüssigkeitsbehältnis auswechselbar im Gehäuse aufnehmbar.

Wenn nämlich der Flüssigkeitsvorrat in einem austauschbar im Gehäuse aufgenommenen Flüssigkeitsbehältnis, beispielsweise in Form einer Zylinderampulle (Karpule) aufgenommen ist, lassen sich mit ein und derselben Vorrichtung mit geringstmöglichen Aufwand nicht nur unterschiedliche Flüssigkeiten injizieren, also beispielsweise flüssige pharmazeutische Präparate unterschiedlicher Art, wie sie bei einer Impfserie benötigt werden können, indem einfach nacheinander Behältnisse mit verschiedenen Flüssigkeiten in die Vorrichtung eingesetzt werden. Die Anordnung hat darüber hinaus den Vorteil, dass sich die Vorrichtung ohne das darin aufgenommene Flüssigkeitsbehältnis besonders einfach und gründlich reinigen und/oder sterilisieren lässt, was insbesondere bei ihrem Einsatz in pharmazeutischen Bereichen, aber auch im (kommerziellen) kosmetischen Bereich von Bedeutung ist.

Wie bereits vorstehend angesprochen, hat es sich als sehr vorteilhaft erwiesen, wenn die Auslassdüse am Flüssigkeitsbehältnis angeordnet ist. Diese Anordnung erlaubt es in besonders einfacher Weise, die Art und Form der Düse, insbesondere den darin vorgesehenen Durchlass für die Flüssigkeit, bestmöglich an die spezifische, im Flüssigkeitsbehältnis aufgenommene und zu injizierende Flüssigkeit anzupassen. Beispielsweise kann es erforderlich sein, bei der Verarbeitung von Flüssigkeiten mit vergleichsweise hoher Viskosität wie beispielsweise Hyaluronsäurepräparaten, die in kosmetischen Anwendungsbereichen z.B. zur Faltenunterspritzung oder zum Modellieren von Lippen und in der Medizin zum Einspritzen in arthrosegeschädigte Gelenke zum Einsatz kommen, eine Düse mit größerem Durchlassquerschnitt vorzusehen als zum Einspritzen von einfacher, physiologischer Kochsalzlösung. Die Anordnung der Auslassdüse unmittelbar am Flüssigkeitsbehältnis stellt dann sicher, dass in jedem Fall die passende Auslassdüse für die jeweilige im Behältnis aufgenommene Flüssigkeit zum Einsatz kommt. Insbesondere aus hygienischen Gründen wird bevorzugt, dass es sich bei den in der erfindungsgemäßen Injektionsvorrichtung zum Einsatz kommenden Flüssigkeitsbehältnissen, insbesondere solchen mit daran angeordneten Auslassdüsen, um Einmal-Behältnisse handelt, die nach einmaliger Verwendung entsorgt, also nicht wieder befüllt werden.

Die Auslassdüse kann einen im Wesentlichen koaxial zur Gehäuseachse des Gehäuses verlaufenden Düsenauslass aufweisen. Die Flüssigkeit tritt dann in einer koaxial zur Gehäuseachse des Gehäuses verlaufenden Richtung aus und damit im Allgemeinen senkrecht auf die Oberfläche des Substrats auf, denn bei der Handhabung des Geräts wird das Gehäuse im Allgemeinen lotrecht zur Substratoberfläche, beispielsweise eine Hautfläche, ausgerichtet. Es ist aber in besonders vorteilhafter Weise auch möglich, dass die Auslassdüse einen Düsenauslass aufweist, der unter einem Winkel zur Gehäuseachse verläuft, wobei der Winkel vorzugsweise größer ist als 45°. Ganz besonders vorteilhaft ist es, wenn der Düsenauslass in einer Richtung liegt, die im Bereich von über 75° bis hin zu einem rechten Winkel (oder sogar darüber hinaus) liegt, die Auslassrichtung also im Wesentlichen in einer Normalebene zur Gehäuseachse des Gehäuses verläuft. Bei im Wesentlichen gleichbleibender, also etwa lotrecht zur Substratoberfläche gewählter Ausrichtung des Gehäuses ermöglicht es diese Ausgestaltung der Erfindung, die Flüssigkeit im Wesentlichen parallel zur Substratoberfläche dicht unter diese in das Substrat zu injizieren, was besonders einfach dann erreichbar ist, wenn das Substrat wie beispielsweise die Haut eines Menschen in seiner/ihrer oberen Schicht nachgiebig ist und mithilfe der Vorrichtung ein Stück weit muldenartig eingedrückt werden kann, so dass sich der Düsenauslass dann in dieser muldenartigen ausbildenden Vertiefung unterhalb des Niveaus des benachbarten Substrats befindet und dann die Flüssigkeit im Wesentlichen parallel zur Substratoberfläche unterhalb von dieser injiziert werden kann. Insbesondere für eine derartige Auslassdüse kann diese oder das vordere Ende des Gehäuses mit einer Tiefenanzeige oder einem Tiefenanschlag versehen sein, so dass die Flüssigkeit genau in die gewünschte Tiefe unterhalb der Substratoberfläche eingebracht werden kann.

Nach der Erfindung ist in vorteilhafter Weise vorgesehen, dass das Flüssigkeitsbehältnis mit der darin aufgenommenen Flüssigkeit zusammen mit einem Ejektionsstößel der Ejektoreinrichtung in dem Gehäuse bzw. einer darin vorgesehenen Beschleunigungsstrecke beweglich aufgenommen ist und dass ein zwischen dem Gehäuse und dem Flüssigkeitsbehältnis wirksamer Anschlag beispielsweise an dem vorderen Auslassende des Gehäuses vorgesehen ist. Die erfindungsgemäße Ausgestaltung hat den Vorteil, dass das Flüssigkeitsbehältnis zusammen mit der darin aufgenommenen Flüssigkeit zunächst gemeinsam mit der Ejektoreinrichtung in dem Gehäuse beschleunigt wird, bevor die Flüssigkeit aus ihrem Behältnis durch die Auslassdüse ausgestoßen wird. Hierdurch wird die Druckerhöhung in der Flüssigkeit bei Betätigung der Ejektoreinrichtung zum Ejizieren der Flüssigkeit begrenzt, indem der Flüssigkeit zunächst eine dynamische Druckkomponente aufgeprägt wird. Besonders bei druckempfindlichen Flüssigkeiten kann hierdurch die Gefahr einer Beschädigung verringert oder ganz vermieden werden. Um den (statischen) Druckanstieg in der Flüssigkeit beim Auftreffen des Flüssigkeitsbehältnisses an dem Anschlag zu verlangsamen, ist es vorteilhaft, wenn ein zwischen dem Anschlag und dem Flüssigkeitsbehältnis wirksamer Anschlagdämpfer, beispielsweise ein Elastomer-Pufferelement vorgesehen ist.

Die Ejektoreinrichtung mit elektromagnetischem Antrieb, der eine eigenständige erfinderische Leistung zukommt eignet sich in ganz vorzüglicher Weise dazu, eine Serie von Injektion in kurzer zeitlicher Abfolge an verschiedenen, bevorzugt unmittelbar zueinander benachbarten Stellen im Substrat zu platzieren. Hierzu wird der Ejektorstößel unmittelbar nach Ejektion einer Teilmenge Flüssigkeit aus dem Flüssigkeitsvorrat, bevorzugt durch kurzzeitiges Umkehren der Stromrichtung in der Spule, wieder zurück in seine Ausgangsstellung verstellt und ist so binnen kürzester Zeit für einen weiteren Injektionsvorgang bereit, zu dem er mittels der elektromagnetischen Spule wieder in Injektionsrichtung beschleunigt wird und erneut für einen Ausstoß einer Flüssigkeitsteilmenge aus dem Flüssigkeitsvorrat erzeugt. Besonders für die Durchführung solcher Serieninjektionen eignet sich ein nach Art einer Zylinderampulle (Karpule) gestaltetes, einen Zylinderraum bildendes Flüssigkeitsbehältnis mit einem an einer Seite vorgesehenen Flüssigkeitsauslass und einem von dem Ejektorstößel betätigbaren Kolben, der in von dem auf ihn auftreffenden Stößel bewirkten Schritten in den Zylinderraum einschiebbar ist, um immer eine Teilmenge Flüssigkeit aus dem Flüssigkeitsauslass auszustoßen, die dann die Vorrichtung durch die Auslassdüse verlässt. Da der Kolben mit jedem Injektionsvorgang von dem Ejektorstößel zunehmend tief in den Zylinderraum des Flüssigkeitsbehältnisses eingeschoben wird, vergrößert sich schrittweise die Beschleunigungsstrecke, die dem Ejektorstößel zwischen einem hinteren, gleichbleibenden Anschlag im Gehäuse und seinem vorderen, vom Kolben definierten Anschlagpunkt zur Verfügung steht. Da die Vergrößerung der Beschleunigungsstrecke bei ansonsten unveränderten Rahmenbedingungen, insbesondere gleichbleibender Stromstärke, mit der der elektromagnetische Antrieb beaufschlagt wird, eine zunehmend größere Geschwindigkeit des Stößels und des zusammen mit diesem bewegten Flüssigkeitsbehältnisses beim Auftreffen an dem Bremsanschlag zur Folge hätte, womit dann auch die der Flüssigkeit eingeprägte Strahlgeschwindigkeit und damit deren Eindringtiefe in das Gewebe ansteigen würden, sind bevorzugt Mittel zur Anpassung der Anfangsgeschwindigkeit des Flüssigkeitsbehältnisses vorgesehen, die es ermöglichen, unabhängig von der Stellung des Kolbens im Flüssigkeitsbehältnis dieses zusammen mit dem darin (noch) enthaltenen Flüssigkeitsvorrat wiederholbar zumindest annähernd auf eine gleich große Anfangsgeschwindigkeit zu beschleunigen, so dass die in Serie erzeugten Injektionen jeweils bis in dieselbe Tiefe in das Substrat eindringen. Die erfindungsgemäße Vorrichtung eignet sich somit in vorteilhafter Weise für das Unterspritzen von Falten in der Haut eines Patienten oder aber auch zur Erzeugung von Tätowierungen, die mit der Erfindung nadellos erzeugt werden können.

Es ist auch möglich, dass sich der Elektromagnet und/oder der zu dessen Betrieb vorgesehene Energiespeicher (Batterie/Akku) an dem beweglichen Teil der Ejektoreinrichtung, also insbesondere dem Ejektorstößel befinden und gemeinsam mit diesem aus dem Gehäuse entfernt werden kann/können, bspw. um dieses vor einem erneuten Geräteeinsatz zu reinigen und/oder zu sterilisieren.

Weitere Merkmale und Vorteile der Erfindung ergeben sich aus der nachfolgenden Beschreibung und der Zeichnung, worin bevorzugte Ausführungsformen der Erfindung anhand von Beispielen dargestellt und näher erläutert sind. Es zeigt:
- Fig. 1: eine Übersichtsdarstellung einer erfindungsgemäßen Injektionsvorrichtung in perspektivischer Ansicht;
- Fig. 2a - c: das Handteil der Injektionsvorrichtung nach Fig. 1 im Längsschnitt in verschiedenen Arbeitsstellungen der Ejektoreinrichtung;
- Fig. 3: die Ejektoreinrichtung mit einer ersten Ausführungsform einer bei der Erfindung zum Einsatz kommenden Auslassdüse, im Längsschnitt;
- Fig. 4: eine zweite Ausführungsform einer Auslassdüse zum Einsatz mit der erfindungsgemäßen Vorrichtung im Schnitt;
- Fig.5: eine dritte Ausführungsform einer Auslassdüse zum Einsatz mit der erfindungsgemäßen Vorrichtung im Schnitt;
- Fig. 6: eine vierte Ausführungsform einer Auslassdüse zum Einsatz mit der erfindungsgemäßen Vorrichtung im Schnitt;
- Fig. 7: die Blendenscheiben des bei der Ausführungsform nach Fig. 6 zum Einsatz kommenden Blendenstapels in einer perspektivischen, auseinander gezogenen Darstellung (Explosionsdarstellung); und
- Fig. 8: eine fünfte Ausführungsform einer Auslassdüse zum Einsatz mit der erfindungsgemäßen Vorrichtung im Schnitt.

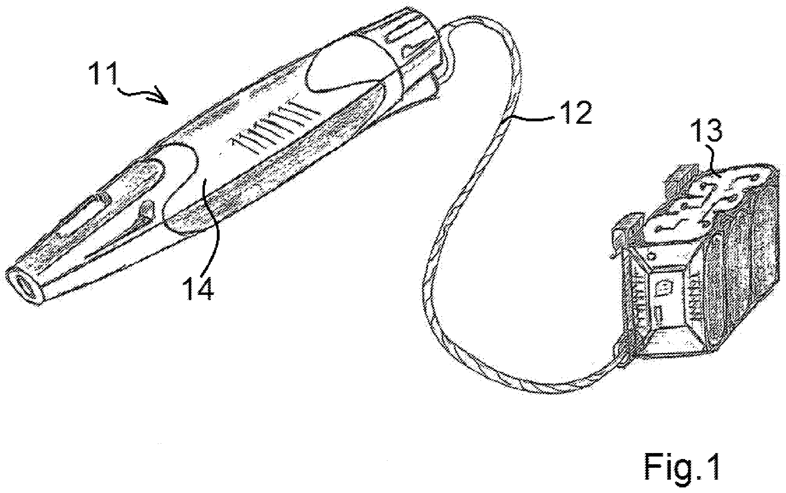

In Fig. 1 ist mit 10 in ihrer Gesamtheit eine Injektionsvorrichtung nach der Erfindung bezeichnet, die ein Handteil 11 aufweist, das über eine Kabelverbindung 12 mit einer externen Stromversorgung 13 , im gezeigten Ausführungsbeispiel einem Akkupack, verbunden ist.

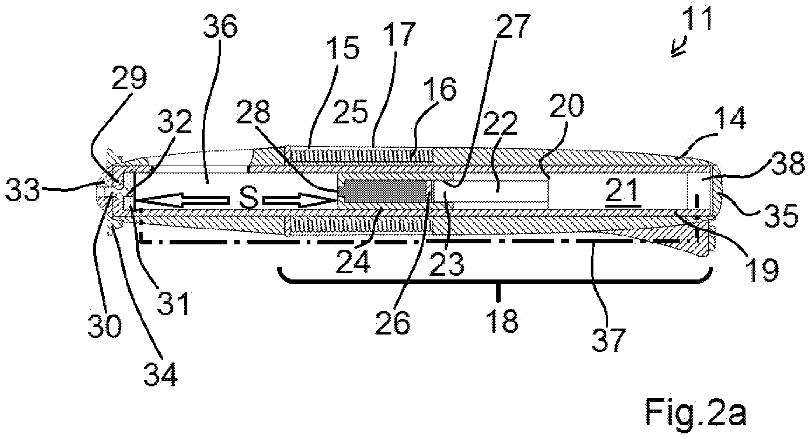

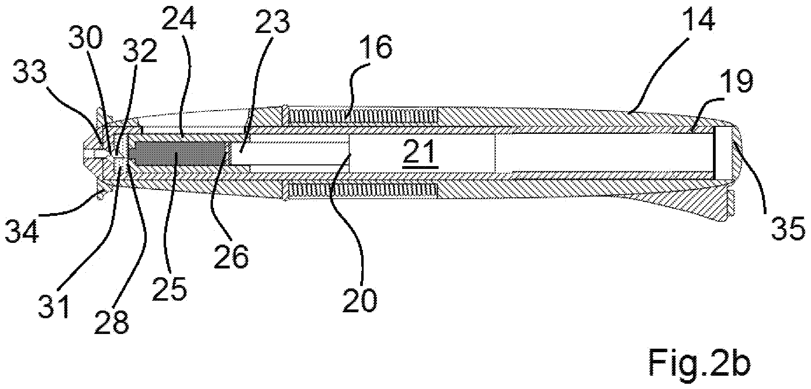

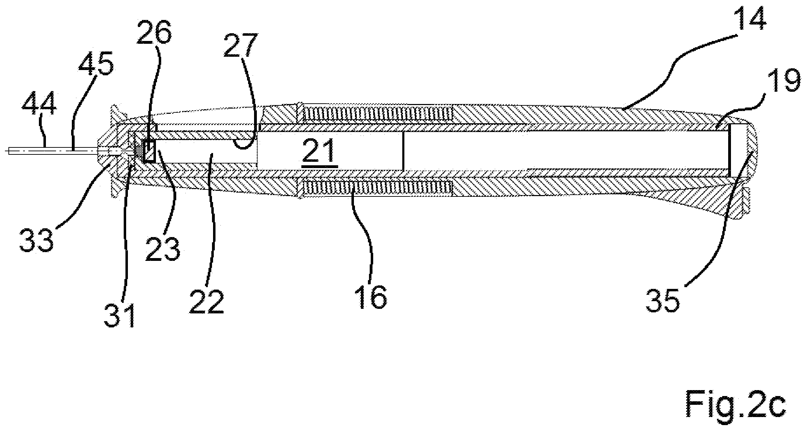

Das Handteil 11 der Injektionsvorrichtung 10 kann von seinem Benutzer bequem mit einer einzigen Hand gehandhabt werden. Der nähere Aufbau des Handteils 11 ist in der Schnittdarstellung gemäß den Fig. 2 a bis c gut zu erkennen. Demnach weist es ein Gehäuse 14 auf, das an seinem Außenumfang mit einer Aussparung 15 versehen ist, in der eine Magnetspule 16 aufgenommen ist. Die Magnetspule 16 ist mittels einer umlaufenden Abdeckung 17 geschützt.

Die Magnetspule 16 ist Bestandteil einer insgesamt mit 18 bezeichneten Ejektoreinrichtung, zu der weiter ein in das Gehäuse eingestecktes, dieses im Wesentlichen von seinem hinteren Ende (in der Zeichnung rechts) bis zu dem vorderen (links) Auslassende durchsetzendes Ejektorrohr 19 aus Kunststoff und ein in diesem längsverschieblich geführter Ejektorstößel 20 gehören, der bei dem gezeigten Ausführungsbeispiel einen hinteren Abschnitt 21 und einen vorderen Abschnitt 22 aufweist. Während der hintere Abschnitt mit größerem Durchmesser an den Innenquerschnitt des Ejektorrohrs 19 angepasst ist und weitestgehend spiel- und reibungsfrei in diesem gleiten kann, weist der vordere Abschnitt 22 einen geringeren Durchmesser auf. Er bildet ein Druckstück 23, das von hinten in ein zylindrisches Flüssigkeitsbehältnis 24 in Form einer Flüssigkeitspatrone oder -kartusche (Karpule) einschiebbar bzw. eingeschoben ist, das eine in ein Substrat, beispielsweise in oder unter die Haut eines Menschen oder Tiers, zu injizierende Flüssigkeit 25 enthält. Dieses Flüssigkeitsbehältnis 24 ist ähnlich wie der hintere Abschnitt 21 des Ejektorstößels 20 in dem Ejektorrohr 19 im Wesentlichen spielfrei aufgenommen, so dass es ebenfalls leicht in diesem gleiten kann. Rückseitig (in der Zeichnung also rechts) ist das Flüssigkeitsbehältnis 24 mit einem Kolben 26 verschlossen, der die Flüssigkeit 25 im Behältnis 24 hält und so weit in den von dem Behältnis 24 definierten Zylinderraum 27 eingeschoben ist, dass an seiner Rückseite das Druckstück 23 ebenfalls ein Stück weit in diesen Zylinderraum einfasst. An seiner in der Zeichnung linken Auslassseite ist das Flüssigkeitsbehältnis 24 mit einer Membran 28 oder einem elastischen Gummistopfen verschlossen.

Das Ejektorrohr 19 ist an seiner vorderen, in Fig.2 linken Stirnseite mit einer Kappe 29 versehen, die eine zentrale Öffnung aufweist, in der eine nach innen in Richtung auf das Flüssigkeitsbehältnis 24 vorspringende Aufstechkanüle 30 aufgenommen ist. Innen an der Kappe ist ein die Aufstechkanüle 30 umgebendes, elastisches Pufferelement 31 angeordnet. Die Aufsteckkanüle 30 hat bei dem gezeigten, bevorzugten Ausführungsbeispiel einen verhältnismäßig großen Durchmesser und damit Durchlassquerschnitt, so dass Flüssigkeit aus dem Flüssigkeitsbehältnis mit nur geringem Druckverlust hindurchströmen kann. Der Innen-Durchmesser der Kanüle 30 kann hierzu beispielsweise ein Viertel bis die Hälfte des Innendurchmessers des Flüssigkeitsbehältnisses betragen.

Die Aufstechkanüle 30 steht mit ihrem ihrer Aufstechspitze 32 gegenüberliegenden, auslassseitigen Ende ein Stück weit über die Kappe 30 vor und bildet hierdurch eine Zentrierung für eine Auslassdüse 33, die auf dieses auslassseitige Ende der Kanüle 30 aufgesteckt und am Gehäuse 14 mittels einer Überwurfmutter 34 festgelegt ist.

Um die Vorrichtung zur Benutzung vorzubereiten, wird zunächst der Ejektorstößel 20 mit seinem vorderen, das Druckstück 23 bildenden Abschnitt 22 von hinten in das patronenartig ausgestaltete Flüssigkeitsbehältnis 24 eingesteckt, wobei sich die Stirnseite des Druckstücks 23 an den Kolben 26 in der zylindrischen Öffnung des Flüssigkeitsbehältnisses anlegt. Diese Baueinheit aus Flüssigkeitspatrone 24 und Ejektorstößel 20 kann dann mit der die Flüssigkeitspatrone vorne verschließenden Membran 28 voran von hinten in das Ejektorrohr 19 im Gehäuse 16 eingeschoben werden, wozu eine rückwärtig am Gehäuse angeordnete Abdeckkappe 35 geöffnet werden kann. Nach Schließen der Abdeckkappe ist die Vorrichtung betriebsbereit. Dieser Betriebszustand ist in Fig. 2a gezeigt.

Anhand der Fig.2a bis 2c lässt sich die Wirkungsweise der erfindungsgemäßen Vorrichtung bei einem Injektionsvorgang gut nachvollziehen: Fig. 2a zeigt dabei die Ausgangsstellung der Ejektoreinrichtung, bei der sich der hintere Abschnitt 21 des Ejektorstößels 20 in der am weitesten hinten gelegenen Position (in der Zeichnung rechts) befindet (hintere Endstellung). Der in dieser Stellung des Ejektorstößels und des vorne auf diesen aufgesteckten Flüssigkeitsbehältnisses vor diesem bis zur endseitigen Kappe 29 sich erstreckende Freiraum in dem Ejektorrohr 19 bildet eine Beschleunigungsstrecke S, auf deren Länge die aus Stößel und Flüssigkeitsbehältnis 24 bestehende Baueinheit beschleunigt werden kann. Um aus der in Fig.2a gezeigten Stellung einen Injektionsvorgang auszulösen, wird die Magnetspule 16 mit elektrischer Energie aus dem Akkupack 13 bestromt und beschleunigt hierdurch den Ejektorstößel 20 mit der daran vorne aufgesteckten Flüssigkeitspatrone 24 über die Beschleunigungsstrecke S in einer Bewegungsrichtung in Richtung auf die Auslassdüse (in der Zeichnung nach links). Dabei erreicht die beschleunigte Baueinheit in kürzester Zeit eine sehr hohe Geschwindigkeit, die in der Praxis über 500 m/s, bei geeignet längerer Beschleunigungsstrecke sogar über 800 m/s betragen kann. Diese Bewegung macht das Flüssigkeitsbehältnis 24 mit der darin aufgenommenen Flüssigkeit 25 zunächst mit, bis es von dem Pufferelement 31 abgebremst wird, das zwischen der vorderen Kappe 29 des Ejektorrohrs 19 und der mit großer Geschwindigkeit von der Magnetspule 16 in Richtung auf die Auslassdüse 33 bewegten Baueinheit aus Ejektorstößel 20 und Flüssigkeitsbehältnis 24 zusammengedrückt wird. Das Pufferelement 31 dient vor allem dazu, zu verhindern, dass das gegen die vordere Kappe 29 anschlagende Flüssigkeitsbehältnis 24 von diesem wieder zurückspringt. Die Stellung der Ejektoreinrichtung in diesem Betriebszustand ist in Fig. 2b gezeigt.

Wie in der Darstellungen gemäß Fig.2a lediglich schematisch in strichpunktierten Linien angedeutet ist, ist der Freiraum 36, der im Inneren des Ejektorrohrs 19 zwischen dessen vorderer Kappe 29 und dem stirnseitigen, von der Membran verschlossenen Ende des Flüssigkeitsbehältnisses 24 vorhanden ist, mittels einer Überströmleitung 37 mit dem Raum 38 hinter dem hinteren Stößelende 21 verbunden. Durch die Überströmleitung kann Luft aus dem vorderen Freiraum 36 verdrängt bzw. tatsächlich aktiv durch den sich hinter dem Stößel bei dessen Vorwärtsbewegung bildenden Unterdruck im Raum 38 abgesaugt werden, womit sichergestellt ist, dass der Ejektorstößel 20 mit dem Flüssigkeitsbehältnis 24 nicht infolge erhöhten Luftwiderstands abgebremst wird. In der praktischen Umsetzung dieses Merkmals kann die Überströmleitung in der Wandung des Gehäuses integriert sein, so dass sie von außen tatsächlich nicht weiter auffällt.

Sobald die Aufstechspitze 32 der Aufstechkanüle 30 die am vorderen Ende des Flüssigkeitsbehältnisses 24 vorgesehene Membran 28 durchsticht, kann die in dem Behältnis aufgenommene Flüssigkeit 25 aus diesem vorne austreten und durch die Kanüle 30 in die Auslassdüse 33 gelangen. Da die Membran 28 des Flüssigkeitsbehältnisses 24 zu einem Zeitpunkt aufgestochen wird, zu dem sich die im Behältnis aufgenommene Flüssigkeit 25 immer noch mit hoher Geschwindigkeit bewegt, kommt zwar die Bewegung des Flüssigkeitsbehältnisses 24 bei dessen Auftreffen am Pufferelement 31 sehr abrupt zum Abbruch, sobald das Pufferelement 31 maximal möglich zusammengepresst ist. Die statische Druckerhöhung in dem im Behältnis 24 aufgenommenen Flüssigkeitsvolumen allerdings bleibt tatsächlich gering, denn die der Flüssigkeit bei der Beschleunigung aufgeprägte Bewegungsenergie wird allenfalls zum Teil in statische Energie (Druckerhöhung) umgesetzt. Vielmehr tritt zumindest eine Teilmenge der im Behältnis enthaltenen Flüssigkeit mit der Anfangsgeschwindigkeit, auf die sie (zusammen mit dem Flüssigkeitsbehältnis) beschleunigt wurde, zunächst in die Kanüle und von dort in die Auslassdüse ein, die sie dann mit einer dem hohen Gesamtdruck (=Summe aus kinetischer und statischer Energie) beim Öffnen des Flüssigkeitsbehältnisses entsprechenden großen Mündungsgeschwindigkeit an der Auslassseite der Auslassdüse verlässt, wo der Flüssigkeit Umgebungsdruck aufgeprägt wird und die ihr innewohnende (Gesamt-)Druckenergie in kinetische Energie (Geschwindigkeit) umgesetzt wird. In der Praxis kann die zum Einsatz gebrachte Auslassdüse, die bevorzugt wie nachstehend noch beschrieben gestaltet ist, einen Durchlass 36 für die Flüssigkeit 25 mit einem Durchmesser von 80 bis 300 µm aufweisen, so dass die ausgestoßene Flüssigkeit als sehr feiner Flüssigkeitsstrahl mit entsprechend geringem Querschnitt auf das Substrat mit sehr großer Geschwindigkeit auftrifft. Die Austrittsgeschwindigkeit der Flüssigkeit kann dabei ohne weiteres 1000 m/s betragen. Mit diesem extrem schnellen und dünnen Flüssigkeitsstrahl wird in dem Substrat ein Injektionskanal bis in eine Tiefe erzeugt (geschossen), die abhängig ist von der Strahlgeschwindigkeit und seinem Durchmesser und damit letztendlich von der Geschwindigkeit, auf die der Ejektorstößel den Flüssigkeitsvorrat beschleunigt hat.

Wenn die gesamte Flüssigkeitsmenge an einem Injektionsort in das Substrat injiziert werden soll, kann hierzu die Magnetspule 16 nach Erreichen der vorderen Endstellung des Flüssigkeitsbehältnisses 24 (Fig. 2b) weiter bestromt werden. Dadurch wird der Ejektorstößel 20 mit dessen vorderen Abschnitt 22 (Druckstück 23) weiter von hinten gegen den Kolben 26 im Flüssigkeitsbehältnis gedrückt, so dass die nach dem Abbau des Drucks im Behältnis durch Ausstoß einer ersten Teilmenge noch verbliebene Flüssigkeit (zweite Teilmenge) wie bei einer herkömmlichen Spritze durch die Kanüle 30 gedrückt und anschließend durch die Auslassdüse 33 ausgestoßen wird. Es hat sich überraschend gezeigt, dass trotz des dann deutlichen geringeren Drucks bzw. der geringeren Geschwindigkeit, mit der die zweite Teilmenge ejiziert wird, diese zuverlässig und vollständig in den mittels der ersten Teilmenge vorab in dem Substrat erzeugten Injektionskanal eindringt und somit in das Substrat, also bei dem Ausführungsbeispiel in oder unter die Haut gelangt. Dabei kommt es im Allgemeinen am Ende des Injektionskanals zu einer Depotbildung, d.h. die zweite Teilmenge Flüssigkeit verteilt sich im Wesentlichen gleichmäßig in dem Gewebe kugelförmig um den Endbereich des Injektionskanals herum. Der Injektionsvorgang kann so lange fortgesetzt werden, bis der Kolben 26 von dem Druckstück 23 vollständig bis zum vorderen Ende des Flüssigkeitsbehältnisses eingeschoben ist (Fig. 2c).

Wenn es gewünscht wird, kann mit der Vorrichtung auch eine Abfolge von mehr oder weniger dicht beieinander positionierten Injektionen von jeweils vergleichsweise kleinen Flüssigkeitsmengen gesetzt werden, und zwar in kurzen zeitlichen Abständen. Hierzu wird durch geeignete Ansteuerung (Änderung der Stromflussrichtung) der Magnetspule 16 der Ejektorstößel 20 direkt nach Erzeugen eines Impulsstoßes in der im Behältnis aufgenommenen Flüssigkeit wieder zurück in seiner Ausgangsstellung gezogen (also in der Zeichnung nach rechts). Da das Flüssigkeitsbehältnis 24 nach dem allerersten, wie vorstehend beschrieben durchgeführten Injektionsvorgang von der Aufstechspitze 32 der Kanüle 30 an der Membran bereits geöffnet ist, kann es bei dieser Betriebsweise in seiner in der Zeichnung gemäß Fig.2c gezeigten linksseitigen Endstellung verbleiben, was durch ein geeignetes, nicht dargestelltes Rückhalteelement gewährleistet werden kann. Beispielsweise kann zu diesem Zweck ein quer zur Längsachse des Ejektorrohrs 19 mit einer Feder radial nach innen vorgespannter Riegel in einer Aussparung im Ejektorrohr aufgenommen sein, der nach dem Vorbeigang der Flüssigkeitspatrone nach Auslösen des ersten Injektionsvorgangs sich unter dem Federdruck radial nach innen bewegt und dabei hinter den rückwärtigen (in der Zeichnungen am rechten Ende des Flüssigkeitsbehältnisses) Rand des Flüssigkeitsbehältnisses fasst und so verhindert, dass sich dieses wieder zurück bewegen kann. Es ist aber ebenfalls möglich, das Flüssigkeitsbehältnis zusammen mit dem Ejektorstößel zurückzuziehen, ggf. auch gemeinsam mit der Aufstechkanüle und der daran auslassseitig anschließenden Auslassdüse. In diesem Fall ist es von Vorteil, wenn anstelle einer dünnen Membran zum Verschließen des Flüssigkeitsbehältnisses ein Gummistopfen an dessen Auslassseite vorgesehen ist, das die Aufstechkanüle mit einer ausreichend großer Haltekraft umschließt, so dass diese beim Zurückziehen sich nicht ohne weiteres von selbst aus dem Stopfen herausziehen kann. Der feste Sitz der Kanüle in dem Verschlussstopfen kann z.B. durch außen an der Kanüle vorgesehene Widerhakenelemente begünstigt werden. Der durch kurzfristige Umpolung der Magnetspule wieder zurückgezogene Ejektorstößel 20 kann in seiner wieder zurückgezogenen Stellung mittels eines kleinen Permanentmagneten oder eines Elektromagneten an der rückwärtigen Abdeckkappe 35 des Gehäuses gehalten werden, so dass er nicht unbeabsichtigt und/oder verfrüht allein infolge seines Eigengewichts wieder gegen das Stoßeinleitungselement (Kolben 26) am Flüssigkeitsbehältnis fällt. Der Ejektorstößel kann dann, ggf. unter Überwindung der Magnethaltekraft des erwähnten (nicht gezeigten) Permanent- oder Elektromagneten, erneut über die vor ihm liegende Beschleunigungsstrecke auf hohe Geschwindigkeit beschleunigt werden, wobei er - im Falle des Verbleibs des Flüssigkeitsbehältnisses am vorderen Ende der Vorrichtung - auf dem Endstück seiner Bewegung mit dem vorderen Druckstück wieder in den Zylinderraum am rückwärtigen Ende des Flüssigkeitsbehältnisses hineingleitet und dort auf dem Kolben 26 aufschlägt und einen Druckstoß zur Ejektion einer weiteren (kleinen) Teilmenge Flüssigkeit erzeugt, oder, - gemäß dem erfindungsgemäßen Verfahren - die Flüssigkeit in dem erneut mit beschleunigten Behältnis 24 ebenfalls wieder auf die Anfangsgeschwindigkeit beschleunigt, mit der diese dann beim Auftreffen des Flüssigkeitsbehältnisses am Anschlagelement ohne nennenswerte statische Druckerhöhung durch die Kanüle und die Auslassdüse ausgestoßen wird.

Die wiederholte Auslösung des Elektromagneten und damit bewirkte Ejektion von Flüssigkeit aus der Vorrichtung kann (nach deren Neupositionierung an der nächsten, gewünschten Injektionsstelle) dabei manuell, also durch Betätigung eines (nicht gezeigten) Auslösemechanismus erfolgen, oder auch automatisch in vorher festgelegten Zeitabständen, wobei diese, beispielsweise bei Verwendung der Vorrichtung als Tätowiergerät, auch sehr kurz gewählt werden können. Ein Betrieb der Vorrichtung mit einer Auslösefrequenz in der Größenordnung von 35 bis 200 Hz ist bei geeigneter Dimensionierung des Stößels und der Beschleunigungsstrecke ohne weiteres möglich.

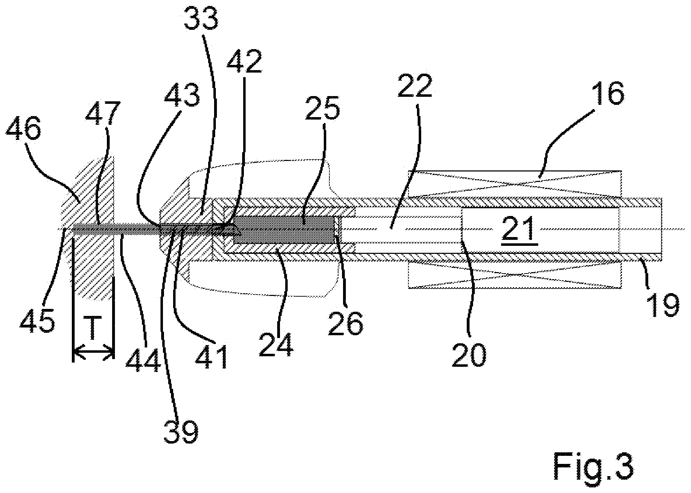

In Fig. 3 ist in ihrem Einbauzustand am Gehäuse der erfindungsgemäßen Vorrichtung eine erste bevorzugte Ausführungsform der zum Einsatz kommenden Auslassdüse 33 dargestellt. Man erkennt, dass diese Auslassdüse 33 einen zentralen, koaxial zu der Kanüle 30 verlaufenden Durchlass 39 für die zu injizierende Flüssigkeit 25 aufweist, der an seiner Durchlasswandung 40 mindestens einen schraubengang- bzw. wendelförmigen Fluidkanal 41 aufweist, der sich vom Düseneinlass 42 an der Seite der Kanüle 30 bis zum Düsenauslass 43 erstreckt, aus dem die Flüssigkeit 25 zur Injektion austritt. Dieser schraubengangförmige Fluidkanal 41 bewirkt, dass der durch die Auslassdüse 33 strömenden Flüssigkeit ein Drall bzw. eine Drehbewegung aufgeprägt wird, so dass der Flüssigkeitsstrahl 44 bei Austritt aus der Düse in Rotation um seine Strahlachse 45 versetzt ist/wird und somit als rotierender Flüssigkeitsstrahl auf das Substrat 46, im Ausführungsbeispiel die Haut eines Menschen oder Tiers auftrifft.

Die Überlagerung der translatorischen Bewegung der Flüssigkeit mit der ihm aufgeprägten Rotation bewirkt, dass sich der Flüssigkeitsstrahl 44 beim Auftreffen auf das Substrat 46 praktisch in dieses hineinschraubt oder hineinbohrt, wobei die schraubenartige Bewegung der Flüssigkeit den Strahl offenbar in sich zusammenhält, so dass es beim Auftreffen auf die Haut- bzw. Substratoberfläche nicht zu einem Aufpilzen und seitlichen Wegspritzen von Flüssigkeit kommt, sondern diese jedenfalls weitestgehend verlustfrei in das Substrat eintritt und in diesem einen Injektionskanal 47 mit einer Tiefe T erzeugt, die im Wesentlichen von der Beschaffenheit des Substrats, der Geschwindigkeit des Flüssigkeitsstrahls in Axialrichtung sowie seinem Querschnitt abhängt. Bei dem gezeigten Ausführungsbeispiel hat der Durchlass 39 in der Auslassdüse auslassseitig einen Durchmesser von ca. 80 bis 100 µm und der infolge des Impulses beim Auftreffen des Flüssigkeitsbehältnisses am Anschlag durch den Düsendurchlass 39 strömende (erste) Teilstrom tritt mit einer Geschwindigkeit in der Größenordnung von 100 bis 1000 m/s aus der Düse aus. Die Tiefe des daraus resultierenden Injektionskanals in (menschlichem oder tierischem) Gewebe kann damit zwischen wenigen Millimetern und einigen Zentimetern eingestellt werden.

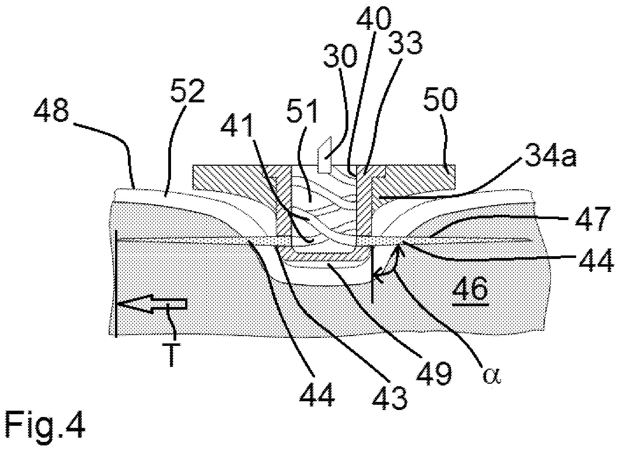

Fig. 4 zeigt eine weitere Ausführungsform einer erfindungsgemäßen Auslassdüse, wobei entsprechende Merkmale mit denselben Bezugszeichen wie bei der ersten Ausführungsform versehen sind. Die in Fig. 4 gezeigte Auslassdüse 33 ist mittels einer Überwurfmutter 34a am Gehäuse festgelegt, die zugleich einen Abstandhalter bzw. eine Tiefenlehre bildet. Die Auslassdüse gemäß Fig.4 kann ein Stück weit in das Substrat 46, nämlich von deren Oberseite 48 in die Haut eines Patienten eingedrückt werden, so dass sie darin einen muldenartige Vertiefung 49 ausbildet. Ein radial nach außen vorspringender Ringbereich 50 an der Überwurfmutter 34a begrenzt dabei die Eindrücktiefe der Düse bzw. zeigt das Erreichen einer gewünschte Tiefe an, was dann der Fall ist, wenn der äußere Rand des Ringbereichs 50 ebenfalls in Kontakt mit der Hautoberfläche 48 gelangt. Die Auslassdüse 33 hat einen Durchlass 39 mit einer einlassseitig etwa tassenförmigen Düsenkammer 51, an deren Wandung zwei (oder mehr) Fluidkanäle 41 ausgebildet sind, die sich nach Art einer Doppel- (oder Mehrfach- )Helix schraubengangförmig umschlingen und die wie beschrieben der durch die Düse strömenden Flüssigkeit einen Drall (Schraubenbewegung) aufprägen. Die Düse hat zwei (oder auch mehrere) seitlich etwa radial nach außen offene Düsenauslässe 43, durch die Flüssigkeitsstrahlen 44 anders als bei der ersten Ausführungsform der Vorrichtung die Düse nicht koaxial zu deren Längsrichtung, sondern in Richtungen verlassen, die im Wesentlichen senkrecht zur Längsachse der Vorrichtung oder - im gezeigten Ausführungsbeispiel - sogar einem Winkel α verlaufen, der geringfügig größer als 90° sein kann. Auf diese Weise ist es leicht möglich, die Flüssigkeit nicht senkrecht zur Substratoberfläche zu injizieren, sondern sie unter die oberste Hautschicht 52 im Wesentlichen parallel zu dieser im Substrat zu verteilen.

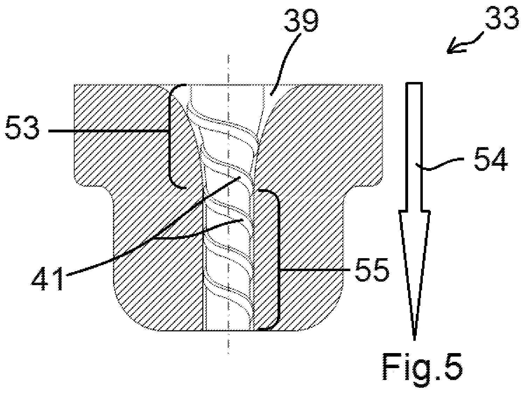

Die in Fig. 5 dargestellte Ausführungsform einer Auslassdüse 33 stimmt weitgehend mit der nach Fig.3 überein. Allerdings hat der Durchlass 39 hier keinen konstanten Querschnitt über seine gesamte Länge, sondern er weist einlassseitig zunächst einen konvergierenden Abschnitt 53 auf, dessen Querschnitt sich in Durchströmrichtung 54 der durch die Düse ejizierten Flüssigkeit 25 verringert, um dann in einen Abschnitt konstanten Querschnitts 55 überzugehen. In beiden Abschnitten 53 und 55 sind an deren Wandungen sich schraubengangförmig wendelnde Fluidkanäle 41 vorgesehen, bei dem gezeigten Ausführungsbeispiel zwei Kanäle, die nach Art einer Doppelhelix angeordnet sind. Der konvergierende Abschnitt sorgt zunächst für eine Beschleunigung der aus dem Flüssigkeitsbehältnis in die Düse gelangenden Flüssigkeit.

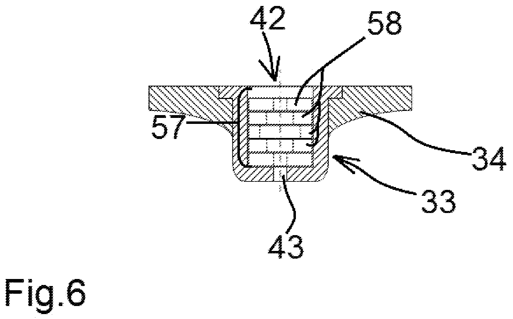

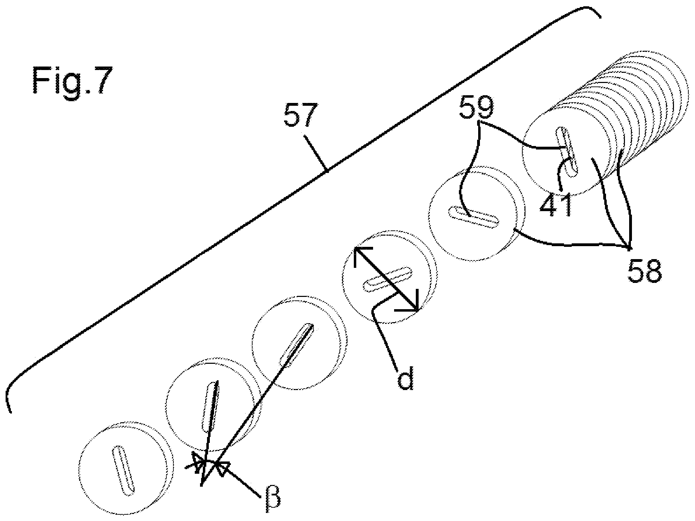

Bei der in den Fig. 6 und 7 gezeigten Ausführungsform hat die Auslassdüse 33 mehrere in Durchlassrichtung 54 der Flüssigkeit hintereinander in Form eines Blendenstapels 57 angeordnete Blendenscheiben 58 aufweist, die jeweils eine sich über einen Teil des Scheibendurchmessers d erstreckende Schlitzöffnung 59 aufweisen, wobei die Schlitzöffnungen 59 von in dem Blendenstapel 57 aufeinander folgenden Blendenscheiben 58 in Umfangsrichtung um einen Winkelbetrag β zueinander versetzt angeordnet sind. Der Betrag dieses Winkelversatzes β in Umfangsrichtung ist an den radial äußeren Enden der Schlitzöffnungen 59 kleiner ist als die Breite der Schlitzöffnungen. Hierdurch ergibt ein wendeltreppenartiger Fluidkanal 41 mit einer zentralen Durchlassöffnung. Die Ausführungsform mit den gestapelten Blenden ist auch mit kleinsten Abmessungen mit einem Durchlassquerschnitt, der im Mikrometerbereich liegt, besonders einfach und kostengünstig herstellbar.

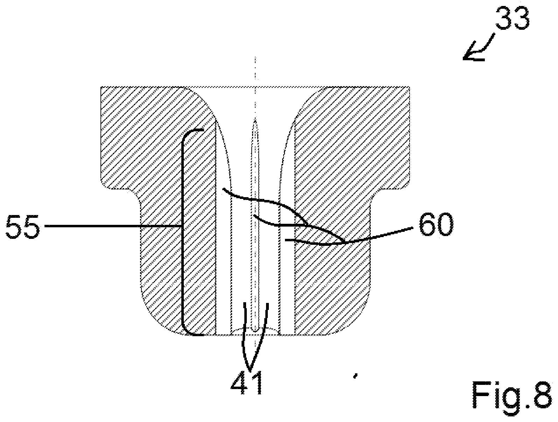

Bei der in Fig. 8 gezeigten Auslassdüse 33 sind an der Wandung 40 des sie durchsetzenden Durchlasses 39 vier Fluidkanäle 41 ausgebildet, die über die Länge des Abschnitts mit konstantem Querschnitt 55 geradlinig parallel zur Durchströmrichtung verlaufen und die durch Stege 60 gegeneinander abgetrennt sind. Die gesamte Düse ist bei dieser Ausführungsform drehbar am Gehäuse der Vorrichtung gelagert und mittels einer Spule elektromotorisch antreibbar. Wenn Sie während des Ejektionsvorgangs in Drehung versetzt wird, übertragen die Stege an der der Durchlasswandung diese Drehbewegung in den äußeren Umfangsbereich des durch die Düse strömenden Flüssigkeitsstrahls und prägen diesem somit die erfindungsgemäße Drehbewegung auf.

Mit der Erfindung wird ein Verfahren zum nadellosen Injizieren von Flüssigkeit in ein Substrat geschaffen, insbesondere zur Injektion von flüssigem, pharmazeutischen oder kosmetischen Präparat in ein biologisches Gewebe, bei dem Flüssigkeit aus einem Fluidvorrat durch eine Auslassdüse ausgestoßen wird und diese als Fluidstrahl verlässt, der in das Substrat eintritt, wobei dieses Verfahren u.a. dadurch ausgezeichnet ist, dass mittels einer mit Hochgeschwindigkeit aus der Auslassdüse austretenden, ersten Teilmenge an Flüssigkeit ein Vorstrahl erzeugt wird, der in dem Substrat einen Injektionskanal ausbildet, und dass anschließend mindestens eine zweite Teilmenge an Flüssigkeit durch den von dem Vorstrahl erzeugten Injektionskanal in das Substrat eingeleitet wird. Dabei wird vorzugsweise die erste Teilmenge Flüssigkeit unter mittels eines Impulsstoßes erzeugtem Hochdruck durch die Auslassdüse ejiziert. In ebenfalls vorteilhafter Ausgestaltung rotiert wenigstens der Vorstrahl bei seinem Eintritt in das Substrat um seine Fluidstrahlachse. Weiterhin kann vorgesehen sein, dass wenigstens die erste Teilmenge bei ihrem Durchgang durch die Auslassdüse in Rotation um die Fluidstrahlachse versetzt wird. Der Impulsstoß wird vorzugsweise mittels eines bevorzugt elektro-magnetisch auf eine Stoßgeschwindigkeit beschleunigten Ejektionsstößels bewirkt, der wenigstens die erste Teilmenge Flüssigkeit mit seiner auf Stoßgeschwindigkeit beschleunigten Masse beaufschlagt. Die mindestens zweite Teilmenge kann mittels des Druck auf die Flüssigkeit ausübenden Ejektionsstößels durch die Auslassdüse ejiziert werden. Dabei kann der Ejektionsstößel zur Druckausübung auf die mindestens zweite Teilmenge mit einer elektro-magnetisch erzeugten Kraft beaufschlagt werden.

Die Erfindung schafft weiter eine Injektionsvorrichtung für die nadellose Injektion einer Flüssigkeit in ein Substrat, insbesondere zur Injektion von flüssigem pharmazeutischen oder kosmetischen Präparat in ein biologisches Gewebe, mit einem Gehäuse, einem im Gehäuse aufgenommenen oder anordbaren Flüssigkeitsvorrat, einer Auslassdüse und mit einer Ejektionseinrichtung zum Ejizieren von Flüssigkeit aus dem Flüssigkeitsvorrat durch die Auslassdüse, wobei die Injektionsvorrichtung dadurch gekennzeichnet ist, dass die Ejektionseinrichtung Mittel zum Erzeugen eines auf wenigstens eine erste Teilmenge an Flüssigkeit im Flüssigkeitsvorrat wirkenden Impulsstoßes aufweist, welche Mittel der Ejektionseinrichtung zum Erzeugen des Impulsstoßes bevorzugt einen auf eine Stoßgeschwindigkeit beschleunigbaren Ejektionsstößel umfassen können, mit dessen auf die Stoßgeschwindigkeit beschleunigter Masse die wenigstens erste Teilmenge Flüssigkeit beaufschlagbar ist. Die Anordnung kann bevorzugt so getroffen sein, dass der Flüssigkeitsvorrat mittels eines von der Ejektionseinrichtung betätigbaren Ejektionskolbens beaufschlagbar ist, welcher wiederum von dem Ejektionsstößel beaufschlagbar ist oder von diesem gebildet werden kann. Die Ejektionseinrichtung kann einen elektromagnetischen Antrieb für den Ejektionsstößel aufweisen.

Die Ejektionseinrichtung weist bevorzugt eine Beschleunigungsstrecke für den Ejektionsstößel auf. Der elektromagnetische Antrieb kann an einem von der Auslassdüse beabstandeten, rückwärtigen Ende des Gehäuses oder etwa in dessen Mitte angeordnet sein und sich die Beschleunigungsstrecke zwischen der Auslassdüse und dem rückwärtigen Gehäuseende erstreckt.

Der elektromagnetische Antrieb kann in ganz bevorzugter Ausgestaltung auch eine am Ejektionsstößel selbst ausgebildete Magnetspule sowie z.B. einen den Ejektionsstößel umgebenden Eisenzylinder aufweisen. Auch kann der Ejektionsstößel mit einer Stromspeichereinrichtung versehen sein, um den Elektromagneten mit Strom zu versorgen.

Die Beschleunigungsstrecke ist vorzugsweise im Bereich vor und hinter dem Ejektionsstößel mit Druckausgleichsöffnungen in Verbindung, wobei diese über eine Überströmleitung miteinander verbunden sein können.

Die Ejektionseinrichtung weist zweckmäßig Mittel zum Erzeugen einer Druckerhöhung im Flüssigkeitsvorrat in unmittelbarem Anschluss an den ausgeübten Impulsstoß auf, welche Mittel zum Erzeugen der Druckerhöhung im Wesentlichen von dem Ejektionsstößel gebildet werden können, der nach Ausübung des Impulsstoßes mittels eines kraftausübenden Antriebs auf den Flüssigkeitsvorrat wirkt. Dabei kann der kraftausübende Antrieb der elektromagnetische Antrieb sein. Der Flüssigkeitsvorrat ist bei der Erfindung vorzugsweise in einem Flüssigkeitsbehältnis aufgenommen ist, das besonders bevorzugt austauschbar im Gehäuse anordbar ist.

Die Ausgestaltung kann auch so getroffen sein, dass die Auslassdüse am Flüssigkeitsbehältnis angeordnet ist. Die Auslassdüse weist vorzugsweise Mittel auf, um den Fluidstrahl wenigstens in seinem Außenbereich vor seinem Auftreffen auf das Substrat in Rotation zu versetzen. Die Auslassdüse kann einen im Wesentlichen koaxial zur Gehäuseachse des Gehäuses verlaufenden Düsenauslass aufweisen. Es ist aber auch möglich, wenn die Auslassdüse einen im Wesentlichen in einer Normalebene zur Gehäuseachse des Gehäuses verlaufenden Düsenauslass aufweist. Die Auslassdüse und/oder das vordere Ende des Gehäuses kann/können mit einen Tiefenanzeige oder einem Tiefenanschlag versehen sein.

In besonders vorteilhafter Weise ist es möglich, dass das Flüssigkeitsbehältnis mit der darin aufgenommenen Flüssigkeit zusammen mit dem Ejektionsstößel in dem Gehäuse bzw. der darin vorgesehenen Beschleunigungsstrecke beweglich aufgenommen ist und das Gehäuse an seinem vorderen Auslassende einen Anschlag für das Flüssigkeitsbehältnis aufweist. Der Anschlag kann mit einem Anschlagdämpfer, beispielsweise einem Elastomer-Puffer Element versehen sein.

Schließlich schafft die Erfindung auch ein Flüssigkeitsbehältnis zur Verwendung bei Ausübung des Verfahrens und/oder in der Vorrichtung das gekennzeichnet ist durch mindestens einen, eine erste, in ein Substrat zu injizierende Flüssigkeit aufnehmenden Kolbenraum, der einen Flüssigkeitsauslass und ein Stoßeinleitungselement zur Einleitung eines auf das Flüssigkeitsbehältnis ausübbaren Impulsstoßes aufweist.

## Patentansprüche

1. Injektionsvorrichtung zum nadellosen Injizieren von Flüssigkeit in ein Substrat, insbesondere eines flüssigen, pharmazeutischen oder kosmetischen Präparats in ein biologisches Gewebe, mit einem Gehäuse (14) zur Aufnahme eines die Flüssigkeit (25) aufnehmenden Flüssigkeitsbehältnisses (24) und mit einer Ejektoreinrichtung (18) zum Ausstoßen der Flüssigkeit (25) aus dem Flüssigkeitsbehältnis (24) durch eine Auslassdüse (33), aufweisend eine Beschleunigungseinrichtung (S;20;16) zum Beschleunigen des Flüssigkeitsbehältnisses (24) mit der darin aufgenommenen Flüssigkeit (25) auf eine Anfangsgeschwindigkeit im Inneren des Gehäuses (14) und eine Verzögerungseinrichtung (29; 30) zum Abbrechen der Bewegung des Flüssigkeitsbehältnisses (24), wobei das Flüssigkeitsbehältnis (24) einschließlich der darin aufgenommenen Flüssigkeit (25) mittels der Beschleunigungseinrichtung (S;20;16) auf die Anfangsgeschwindigkeit beschleunigt ist, bevor die Bewegung des Flüssigkeitsbehältnisses (24) von der Verzögerungseinrichtung (29; 30) abgebremst wird, wobei die Bewegung der im Behältnis (24) enthaltenen Flüssigkeit (25) zumindest teilweise aufrecht erhalten ist, so dass die Flüssigkeit (25) das Flüssigkeitsbehältnis (24) verlässt, durch die Auslassdüse (33) austritt und in dem Substrat einen Injektionskanal erzeugt,
**dadurch gekennzeichnet, dass** die Ejektoreinrichtung (18) einen elektromagnetisch, chemisch und/oder Gas betriebenen Antrieb (16) aufweist, und,
dass das Flüssigkeitsbehältnis (24) auswechselbar im Gehäuse (14) aufnehmbar ist.

2. Injektionsvorrichtung nach Anspruch 1, **dadurch gekennzeichnet, dass** die Beschleunigungseinrichtung (S;20;16) ein an einer der Auslassdüse (33) entgegengesetzten Rückseite (26) des Flüssigkeitsbehältnisses (25) angeordnetes und/oder angreifendes Druckstück (23) umfasst, das mittels der Ejektoreinrichtung (18) gemeinsam mit dem Flüssigkeitsbehältnis (24) und der darin aufgenommenen Flüssigkeit (25) auf die Anfangsgeschwindigkeit im Gehäuse (14) beschleunigbar ist.

3. Injektionsvorrichtung nach Anspruch 1 oder 2, **dadurch gekennzeichnet, dass** die Verzögerungseinrichtung im Wesentlichen von einem zwischen dem Gehäuse (14) und dem Flüssigkeitsbehältnis (24) wirksamen Anschlag (29) gebildet wird.

4. Injektionsvorrichtung nach Anspruch 3, **dadurch gekennzeichnet, dass** der Anschlag (29) mit einem Anschlagdämpfer (31), beispielsweise einem Elastomer-Puffer Element versehen ist.

5. Injektionsvorrichtung nach einem der Ansprüche 1 bis 4, **dadurch gekennzeichnet, dass** das Flüssigkeitsbehältnis (24) einen die Flüssigkeit (25) aufnehmenden Zylinderraum (27) aufweist, in den das Druckstück (23) einschiebbar ist.

6. Injektionsvorrichtung nach einem der Ansprüche 1 bis 5, **dadurch gekennzeichnet, dass** das Flüssigkeitsbehältnis (24) einen an einen Einlassquerschnitt der Auslassdüse (33) angepassten Behälterauslass aufweist.

7. Injektionsvorrichtung nach einem der Ansprüche 1 bis 6, **dadurch gekennzeichnet, dass** das Flüssigkeitsbehältnis an seiner Auslassseite mit einer Membran (28) oder einem Durchstechstopfen verschlossen ist, der von einer Hohlnadel (30) zur Öffnung des Flüssigkeitsbehältnisses durchstechbar ist.

8. Injektionsvorrichtung nach Anspruch 7, **dadurch gekennzeichnet, dass** die Hohlnadel (30) einlassseitig an der Auslassdüse (33) vorgesehen ist.

9. Injektionsvorrichtung nach Anspruch 7 oder 8, **dadurch gekennzeichnet, dass** die Hohlnadel (30) einstückig mit der Auslassdüse ausgestaltet ist oder diese selbst bildet.

10. Injektionsvorrichtung nach einem der Ansprüche 6 bis 9, **dadurch gekennzeichnet, dass** der Behälterauslass eine vorzugsweise vom Querschnitt des Zylinderraums (27) hin zum Einlassquerschnitt der Auslassdüse (33) konvergierende Beschleunigungszone aufweist.

11. Injektionsvorrichtung nach einem der Ansprüche 1 bis 10, **dadurch gekennzeichnet, dass** die Auslassdüse (33) integral oder auswechselbar am Flüssigkeitsbehältnis (24) angeordnet oder anordbar ist.

## Claims

1. Injection device for needleless injection of liquid into a substrate, in particular of a liquid, pharmaceutical or cosmetic preparation into a biological tissue, having a housing (14) for receiving a liquid container (24) receiving the liquid (25) and having an ejector device (18) for ejecting the liquid (25) from the liquid container (24) through an outlet nozzle (33), having an acceleration device (S; 20; 16) for accelerating the liquid container (24) with the liquid (25) received therein to an initial speed in the interior of the housing (14) and a delay device (29; 30) for interrupting the movement of the liquid container (24), wherein the liquid container (24) including the liquid (25) received therein is accelerated to the initial speed by means of the acceleration device (S; 20; 16) before the movement of the liquid container (24) is decelerated by the delay device (29; 30), wherein the movement of the liquid (25) contained in the container (24) is at least partially maintained so that the liquid (25) leaves the liquid container (24), exits through the outlet nozzle (33) and generates an injection channel in the substrate,
**characterized in that** the ejector device (18) has an electromagnetically, chemically and/or gas-operated drive (16), and
**in that** the liquid container (24) can be replaceably received in the housing (14).

2. Injection device according to claim 1, **characterized in that** the acceleration device (S; 20; 16) comprises a pressure piece (23) which is arranged and/or acts on a rear side (26), opposite the outlet nozzle (33), of the liquid container (25) and can be accelerated to the initial speed in the housing (14) by means of the ejector device (18) together with the liquid container (24) and the liquid (25) received therein.

3. Injection device according to claim 1 or 2, **characterized in that** the delay device is formed substantially by a stop (29) which acts between the housing (14) and the liquid container (24).

4. Injection device according to claim 3, **characterized in that** the stop (29) is provided with a stop damper (31), for example an elastomer buffer element.

5. Injection device according to one of claims 1 to 4, **characterized in that** the liquid container (24) has a cylinder chamber (27) which receives the liquid (25) and into which the pressure piece (23) can be pushed.

6. Injection device according to one of claims 1 to 5, **characterized in that** the liquid container (24) has a container outlet adapted to an inlet cross section of the outlet nozzle (33).

7. Injection device according to one of claims 1 to 6, **characterized in that** the liquid container is closed on its outlet side by a membrane (28) or a piercing plug which can be pierced by a hollow needle (30) in order to open the liquid container.

8. Injection device according to claim 7, **characterized in that** the hollow needle (30) is provided on the inlet side of the outlet nozzle (33).

9. Injection device according to claim 7 or 8, **characterized in that** the hollow needle (30) is configured in one piece with the outlet nozzle or forms the latter itself.

10. Injection device according to one of claims 6 to 9, **characterized in that** the container outlet has an acceleration zone which preferably converges from the cross section of the cylinder chamber (27) towards the inlet cross section of the outlet nozzle (33).

11. Injection device according to one of claims 1 to 10, **characterized in that** the outlet nozzle (33) is arranged or can be arranged integrally or replaceably on the liquid container (24).

## Revendications

1. Dispositif d'injection pour l'injection sans aiguille de liquide dans un substrat, en particulier d'une préparation liquide, pharmaceutique ou cosmétique dans un tissu biologique, comprenant un boîtier (14) pour recevoir un récipient de liquide (24) recevant le liquide (25) et comprenant un dispositif éjecteur (18) pour éjecter le liquide (25) hors du récipient de liquide (24) à travers une buse de sortie (33), présentant un dispositif d'accélération (S ; 20 ; 16) pour accélérer le récipient de liquide (24) avec le liquide (25) reçu dans celui-ci à une vitesse initiale à l'intérieur du boîtier (14) et un dispositif de retardement (29 ; 30) pour interrompre le mouvement du récipient de liquide (24), le récipient de liquide (24) y compris le liquide (25) reçu dans celui-ci étant accéléré au moyen du dispositif d'accélération (S ; 20 ; 16) à la vitesse initiale avant que le mouvement du récipient de liquide (24) ne soit freiné par le dispositif de retardement (29 ; 30), le mouvement du liquide (25) contenu dans le récipient (24) étant au moins partiellement maintenu de sorte que le liquide (25) quitte le récipient de liquide (24), sort à travers la buse de sortie (33) et génère dans le substrat un canal d'injection,
**caractérisé en ce que** le dispositif éjecteur (18) présente un entraînement (16) fonctionnant de manière électromagnétique, chimique et/ou gazeuse, et
**en ce que** le récipient de liquide (24) peut être reçu de manière remplaçable dans le boîtier (14).

2. Dispositif d'injection selon la revendication 1, **caractérisé en ce que** le dispositif d'accélération (S ; 20 ; 16) comprend une pièce de pression (23) disposée et/ou agissant sur un côté arrière (26) du récipient de liquide (25) opposé à la buse de sortie (33), qui peut être accélérée au moyen du dispositif éjecteur (18) conjointement avec le récipient de liquide (24) et le liquide (25) reçu dans celui-ci à la vitesse initiale dans le boîtier (14).

3. Dispositif d'injection selon la revendication 1 ou 2, **caractérisé en ce que** le dispositif de retardement est formé essentiellement par une butée (29) agissant entre le boîtier (14) et le récipient de liquide (24).

4. Dispositif d'injection selon la revendication 3, **caractérisé en ce que** la butée (29) est pourvue d'un amortisseur de butée (31), par exemple d'un élément tampon élastomère.

5. Dispositif d'injection selon l'une quelconque des revendications 1 à 4, **caractérisé en ce que** le récipient de liquide (24) présente un espace cylindrique (27) recevant le liquide (25), dans lequel la pièce de pression (23) peut être insérée.

6. Dispositif d'injection selon l'une quelconque des revendications 1 à 5, **caractérisé en ce que** le récipient de liquide (24) présente une sortie de récipient adaptée à une section transversale d'entrée de la buse de sortie (33).

7. Dispositif d'injection selon l'une quelconque des revendications 1 à 6, **caractérisé en ce que** le récipient de liquide est fermé sur son côté de sortie avec une membrane (28) ou un bouchon de perçage, qui peut être percé par une aiguille creuse (30) pour l'ouverture du récipient de liquide.

8. Dispositif d'injection selon la revendication 7, **caractérisé en ce que** l'aiguille creuse (30) est prévue côté entrée sur la buse de sortie (33).

9. Dispositif d'injection selon la revendication 7 ou 8, **caractérisé en ce que** l'aiguille creuse (30) est configurée d'une seule pièce avec la buse de sortie ou forme celle-ci elle-même.

10. Dispositif d'injection selon l'une quelconque des revendications 6 à 9, **caractérisé en ce que** la sortie de récipient présente une zone d'accélération convergeant de préférence de la section transversale de l'espace cylindrique (27) vers la section transversale d'entrée de la buse de sortie (33).

11. Dispositif d'injection selon l'une quelconque des revendications 1 à 10, **caractérisé en ce que** la buse de sortie (33) est disposée ou peut être disposée d'une seule pièce ou de manière remplaçable sur le récipient de liquide (24).
